# EUROPEAN PATENT SPECIFICATION

(11) **EP 0 256 036 B1**
(45) Date of publication and mention of the grant of the patent: **07.04.1993**
(21) Application number: 87900613.8
(22) Date of filing: 31.12.1986
(51) Int. Cl.: A61K 31/40, C07D 487/22

(54) **PRODUCTION AND USE OF PURPURINS, CHLORINS AND PURPURIN- AND CHLORIN-CONTAINING COMPOSITIONS**
HERSTELLUNG UND VERWENDUNG VON PURPURINEN, CHLORINEN UND PURPURIN UND CHLORIN ENTHALTENDEN ZUBEREITUNGEN
PRODUCTION ET UTILISATION DE PURPURINES, CHLORINES ET DE COMPOSITIONS CONTENANT DE LA PURPURINE ET DE LA CHLORINE

(30) Priority: 02.01.1986 US 815714; 18.03.1986 US 842125
(43) Date of publication of application: 24.02.1988
(73) Proprietor: THE UNIVERSITY OF TOLEDO, Toledo, OH 43606 (US); THE MEDICAL COLLEGE OF OHIO, Toledo, OH 43699 (US); SAINT VINCENT MEDICAL CENTER, Toledo, OH 43608 (US)
(72) Inventor: MORGAN, Alan, R., Toledo, OH 43612 (US); SELMAN, Steven, H., Toledo, OH 43615 (US); KREIMER-BIRNBAUM, Martha, Toledo, OH 43606 (US)
(74) Representative: König, Werner, Dipl.-Ing.
(86) International application number: US8602824
(87) International publication number: WO8704071

(56) References cited:
- EP-A- 0 142 732
- WO-A-84/01382
- US-A- 4 604 241
- THE JOURNAL OF ORGANIC CHEMISTRY, vol. 51, no. 8, 18th April 1986, pages 1347-1350, The American Chemical Society; R. MORGAN et al.: "Observations on the synthesis and spectroscopic characteristics of purpurins"
- SURG. FORUM, vol. 37, 1986, pages 659-61; S.H. SELMAN et al.: "Photodynamic therapy of transplantable fanft-induced urothelial tumors using the purpurin NT2 and light"
- CANCER RESEARCH, vol. 48, no. 1, 1st January 1988, pages 194-198, A.R. MORGAN et al.: "New photosensitizers for photodynamic therapy: combined effect of metallopurpurin derivatives and light on transplantable bladder tumors"
- PORPHYRIN PHOTOSENSITIZATION, 1983, pages 3-13, Plenum Publishing Corp., New York, US; D. KESSEL and T.J. DOUGHERTY et al.: "Photoradiation therapy - clinical and drug advances"
- Journal of The American Chemical Society, vol. 82, no. 14, issued 20 July 1960 (Washington, D.C.) "The Total Synthesis of Chlorophyll", pages 3800-3802. See formula V, page 3801.
- Angewandte Chemie International Edit., vol. 14, no. 5, issued May 1975, Academic Press (New York) "Preparation and Reactivity of Sterically Crowded Porphyrins", pages 361-363. See formula (4), page 362.
- Journal of the Chemical Society, Perkin Transactions I,issued 1979(London), "Wittig Condensation Products from Nickel meso-Formyl-octaethyl-phorphyrin and aetioporphyrin I and Some Cyclisation Reactions", pages 1660-1670. See formula 6, on page 1662

## Description

This invention relates to the production and use of a family of purpurins, a family of chlorins and metal complexes of the purpurins and chlorins, and to purpurin- and chlorin-containing compositions. The purpurins have an unsaturated isocyclic ring fused to a reduced pyrrole ring; the unsaturated isocyclic ring of the purpurins corresponds with a saturated ring in the chlorins. The chlorins are useful as green dyes. The chlorins and the purpurins are useful in the detection and treatment of tumors; after they have been administered systemically, e.g., intravenously, they localize preferentially in a tumor. After they have been administered, and have localized in a tumour, their presence can be detected by illumination with ultra violet light, which causes them to fluoresce. The chlorins and purpurins of the invention can also be used to treat tumors; after they have been administered and have localized, irradiation with light of a wave length at which they show an absorbance peak causes a reaction which damages or destroys the tumor where they have localized. The purpurin- and chlorin-containing compositions are solutions thereof in an organic liquid that is physiologically acceptable for intravenous administration and emulsions thereof in saline solutions.

Four purpurins of the family to which the instant invention relates, and having an unsaturated isocyclic ring fused to a reduced pyrrole ring, are known to be reported in the prior art, a communication to the editor by Woodward et al., JACS, Vol. 82, pp. 38,0 et seq., 1960, where they are disclosed as intermediates in the synthesis of chlorophyll, and journal articles by Witte et al., Angew, Chem.Internat. Edit./Vol. 14, No. 5, pp 361 et seq., 1975, and Arnold et al., Journal of the Chemical Society, Perkin Transactions I, pp. 1660 et seq., 1979. No utility for purpurins is suggested by either Witte et al. or Arnold et al. In addition, European patent application EP142,732 is said (C.A. 103: 123271S) to disclose certain chlorins of a different family and that they accumulate preferentially in the cancer cells of hamsters infected with pancreatic cancer.

Purpurins and chlorins are similar in structure to porphyrins. One porphyrin called protoporphyrin IX, can be separated from blood. Hematoporphyrin can be produced from protoporphyrin IX; a chemical mixture derived from hematoporphyrin, called hematoporphyrin derivative, and often abreviated "HpD", can be administered intravenously and used in the manner described above for the detection and treatment of tumors. The exact composition of HpD, however, is not known; in fact, it is a mixture of many different porphyrins and related compounds (see, for example, Porphyrin Photosensitization, edited by David Kassel and Thomas J. Dougherty, Plenum Press, New York and London, 1983, pp.3-13). As a consequence, the chlorins and purpurins of the instant invention are preferred over HpD for this use because they are single, known compounds. In addition, the chlorins and purpurins have absorbance peaks at longer wavelengths and show greater absorbances, by comparison with HpD; the longer wavelength peaks are advantageous because light of the longer wavelengths is capable of greater penetration of tissue, while the greater absorbances are desirable because less light energy is required to cause a given degree of reaction.

### Brief description of the drawings

Fig. 1 is a structural formula for metal complexes of the family of purpurins according to the invention where an unsaturated isocyclic ring is fused to a reduced pyrrole ring.

Fig. 2 is a structural formula for metal complexes of the family of chlorins according to the invention where the isocyclic ring which corresponds with the unsaturated isocyclic ring of the purpurins is saturated.

Fig. 3 is a structural formula for a family of porphyrins which can be used to produce purpurins according to the invention.

Fig. 4 is a structural formula for metal complexes of porphyrins having the formula of Fig. 3.

Fig. 5 is a structural formula for the family of purpurins according to the invention.

Fig. 6 is a structural formula for the family of chlorins according to the invention.

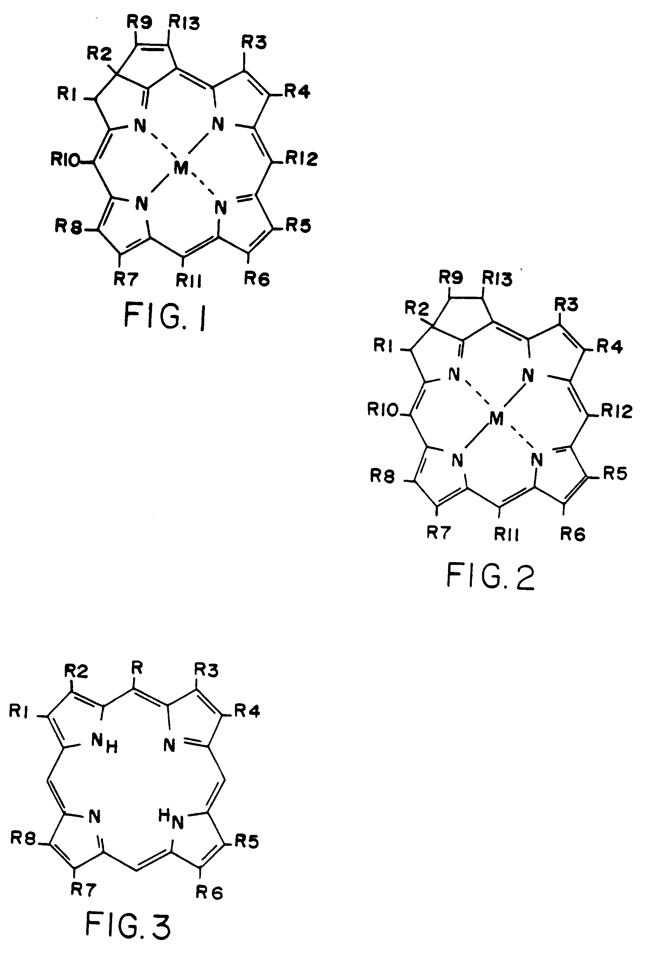

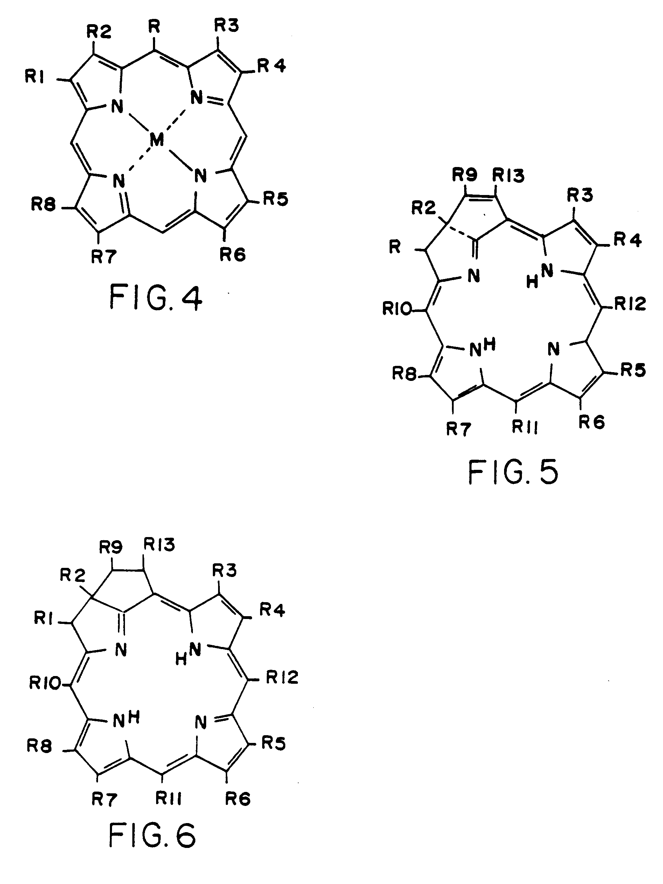

The instant invention, in one aspect, is a solution in an organic/solvent of a purpurin having the structure of Fig. 1 or 5 or of a chlorin having the structure of Fig. 2 or 6 of the attached drawings, where M is a metal, for example, Ag, Al, Ce, Co, Cr, Cu, Dy, Er, Eu, Fe, Ga, Gd, Hf, Ho, In, La, Lu, Mn, Mo, Nd, Ni, Pb, Pd, Pr, Pt, Rh, Sb, Sc, Sm, Sn, Tb, Th, Ti, Tl, Tm, U, V, Y, Yb, Zn or Zr, and each of R1 to R13 is:
H or CHO,
a primary or secondary alkyl group having from 1 to 4 carbon atoms,
an alkylene group having from 2 to 4 carbon atoms,
a group having the formula R₂N(R₃)₂ where R₂ is a bivalent aliphatic hydrocarbon radical having from 1 to 4 carbon atoms, wherein any carbon to carbon bond is either a single or a double bond, and not more than one is a double bond; R₃ is hydrogen or an alkyl radical having from 1 to 2 carbon atoms and the two R₃ groups can be the same or different,
a group having the formula R₂N(R₄)₃⁺ where R₂ is a bivalent aliphatic hydrocarbon radical having from 1 to 4 carbon atoms, wherein any carbon to carbon bond is either a single or a double bond, and not more than one is a double bond; and R₄ is an alkyl group having from 1 to 2 carbon atoms and the three R₄ groups can be the same or different,
a group having the formula R₂OH were R₂ is a bivalent aliphatic hydrocarbon radical having from 1 to 4 carbon atoms, wherein any carbon to carbon bond is either a single or a double bond, and not more than one is a double bond, or
CO₂R', CH₂CO₂R' or CH₂CH₂CO₂R' where R' is H, or a primary or secondary alkyl group having from one to four carbon atoms.
In addition, R1 can be a bivalent aliphatic hydrocarbon radical having from 2 to 4 carbon atoms wherein both of the valences of the radical are attached to the same carbon atom thereof and to a carbon atom of the purpurin, chlorin, or metal complex.
The organic liquid is one in which the purpurin is soluble and which is physiologically acceptable for intravenous or topical administration.

In another aspect, the invention is a chlorin having the structure of Fig. 2 or 6 of the attached drawings where R1 through R13 and M have the meanings set forth above.

In still another aspect, the invention is a family of purpurins having the structure of rig. 1 or 5 of the attached drawings where R1 through R13 and M have the meanings set forth above, except that R' is hydrogen or a primary or secondary alkyl group Raving from 2 to 4 carbon atoms.

A method for detecting and treating tumors comprises administering an effective amount of a purpurin or chlorin to a human or animal patient, and irradiating the relevant region of the patient with ultra violet or visible light of a wavelength at which the purpurin or chlorin has an absorbance peak. The purpurin is one having the structure of Fig. 1 or Fig. 5 while the chlorin is one having the structure of Fig. 2 or Fig. 6 of the attached drawings where M is a metal and each of R1 to R13 is
H or CHO,
a primary or secondary alkyl group having from 1 to 4 carbon atoms,
an alkylene group having from 2 to 4 carbon atoms,
a group having the formula R₂N(R₃ )₂ where R₂ is a bivalent aliphatic hydrocarbon radical having from 1 to 4 carbon atoms, wherein any carbon to carbon bond is either a single or a double bond, and not more than one is a double bond; R₃ is hydrogen or an alkyl radical having from 1 to 2 carbon atoms and the two R₃ groups can be the same or different,
a group having the formula R₂N(R₄)₃⁺ where R₂ is a bivalent aliphatic hydrocarbon radical having from 1 to 4 carbon atoms, wherein any carbon to carbon bond is either a single or a double bond, and not more than one is a double bond; and R₄ is an alkyl group having from 1 to 2 carbon atoms and the three R₄ groups can be the same or different,
a group having the formula R₂OH were R₂ is a bivalent aliphatic hydrocarbon radical having from 1 to 4 carbon atoms, wherein any carbon to carbon bond is either a single or a double bond, and not more than one is a double bond, or
CO₂R', CH₂CO₂R' or CH₂CH₂CO₂R' where R' is H, or a primary or secondary alkyl group having from one to four carbon atoms.
In addition, R1 can be a bivalent aliphatic hydrocarbon radical having from 2 to 4 carbon atoms wherein both of the valences of the radical are attached to the same carbon atom thereof and to a carbon atom of the purpurin, chlorin, or metal complex.

It is therefore an object of the invention to provide a new composition which is a solution in an organic solvent of a purpurin having the structure of Fig. 1 or Fig. 5 or a chlorin having the structure of Fig. 2 or Fig. 6 of the attached drawings.

It is a further object to provide an aqueous emulsion of a solution in an organic liquid of a purpurin having the structure of Fig. 1 or Fig. 5 or of a chlorin having the structure of Fig. 2 or Fig. 6 of the attached drawings.

It is another object to provide a family of purpurins having the structure of Fig. 1 or Fig. 5 of the attached drawings.

It is still another object to provide a family of chlorins having the structure of Fig. 2 or Fig. 6 of the attached drawings.

### Description of the preferred embodiments

Examples 1 through 8 hereof set forth the best mode presently contemplated by the inventors, insofar as this invention is directed to purpurins and chlorins and their production. The in vivo test procedures describe the best mode insofar as this invention is directed to solutions of the purpurins and chlorins in an organic liquid and to the production of such solutions, and the in vitro and in vivo test procedures describe the best mode insofar as purpurins and chlorins are used for the detection and treatment of tumors.

### Example 1

The production of a novel purpurin according to the invention (hereafter "Purpurin NT2") from nickel meso-formyl octaethyl porphyrin is described in this example. The production of nickel meso-formyl octaethyl porphyrin is described in a journal article by R. Grigg et al., J. Chem.Soc. Perkin Trans I, 1972, pp. 1789,1798; it has the formula of Fig. 4 of the attached drawings where R1 through R8 are ethyl, R is CHO, and M is Ni. Two intermediates were produced in the Example 1 procedure, nickel meso-(β-ethoxycarbonylvinyl)octaethyl porphyrin which has the formula of Fig. 4 of the attached drawings where R1 through R8 are ethyl, R is CH=CHCO₂CH₂CH₃, and M is Ni, and meso(β-ethoxycarbonylvinyl)octaethyl porphyrin, which has the formula of Fig. 3 of the attached drawings where R1 through R8 are ethyl, and R is CH=CHCO₂ CH₂CH₃. Purpurin NT2 has the formula of Fig. 5 of the attached drawings where R1 to R8 are ethyl, R9 is CO₂CH₂CH₃, and R10 to R13 are hydrogen.

### Production of nickel meso-(β-ethoxycarbonylvinyl)octaethyl porphyrin

A solution of 506 mg nickel meso-formyl octaethyl porphyrin and 1.024 g (carbethoxymethylene)triphenylphosphorane in 50 ml xylene was heated under reflux for 18 hours. The solution was cooled; the xylene was removed in vacuo; and the solid which remained was dissolved in the minimum amount of dichloromethane and chromatographed on silica. A minor fraction of nickel octaethyl porphyrin and a major red fraction were recovered. The solvent was removed from the red fraction; the solid which remained was recrystallized from a solvent composed of equal parts by volume of dichloromethane and methanol, yielding 455 mg small brown needles. The product was identified by nuclear magnetic resonance as nickel meso-(β-ethoxycarbonylvinyl)octaethyl porphyrin; it showed visible spectrum absorbance peaks at 405, 530 and 565 nm (ε 94 180, 18 604, 27 790).

### Production of Meso-(β-ethoxycarbonylvinyl) octaetheyl porphyrin

A solution was prepared by dissolving 621 mg nickel meso-(β-ethoxycarbonylvinyl)octaethyl porphyrin in 10 ml concentrated (96.7 percent w/w) sulfuric acid; after the solution stood for 2 hours at room temperature of about 22°, an addition of 100 ml dichloromethane was made thereto, followed by saturated aqueous sodium bicarbonate to neutralize the sulfuric acid. The organic layer was collected, washed and dried; the solvent was then vaporized. The crude product which remained was recrystallized from a solvent composed of equal parts by volume of dichloromethane and methanol, yielding 552 mg small reddish-brown crystals which were identified by nuclear magnetic resonance as meso-(β-ethoxycarbonylvinyl)octaethyl porphyrin. The production of this porphyrin is disclosed in a Journal article by Fuhrhop et al., Ann. Chem., 1976, pp. 1539-1559.

### Production of Purpurin NT2

A solution of 100 mg meso-(β-ethoxycarbonylvinyl)octaethyl porphyrin in 20 ml glacial acetic acid was heated under reflux in a nitrogen atmosphere for 24 hours. The solution was then cooled; the acetic acid was removed in vacuo; and the remaining product was dissolved in the minimum amount of dichloromethane and chromatographed on silica, yielding a major green fraction from which the solvent was removed. The solid which remained was recrystallized from 50 % v/v dichloromethane and methanol yielding 68 mg purple microcrystals which were identified by nuclear magnetic resonance as Purpurin NT2, and found to have visible spectrum absorbance peaks at 433, 453, 503, 530, 568, 648 and 695 nm (ε 89 509, 89 509, 14 571, 12 143, 18 908, 10 582, 42 673).

### Example 2

### Production of Zn Purpurin NT2

A solution was prepared by dissolving 20 mg Purpurin NT2 in a mixed solvent composed of 15 ml dichloromethane and 5 ml methanol and 100 mg zinc acetate was added to the solution; the mixture which resulted was refluxed for about 4 minutes until the electronic spectrum of the reaction mixture indicated that chelation was complete. The reaction mixture was then concentrated to 7 ml and allowed to cool to room temperature of about 22°. Product which precipitated was recovered by filtration, dissolved in a mixed solvent composed of 5 ml dichloromethane and 2 ml methanol, and recrystallized, yielding 18 mg Zn Purpurin NT2 in the form of microcrystals. The Zn Purpurin NT2, a metal complex, has the formula of Fig. 1 of the attached drawings where R1 to R8 are ethyl, R9 is CO₂CH₂CH₃ , R10 to R13 are hydrogen and M is Zn; the compound has visible spectrum absorbance peaks at 413, 435, 535, 578, 618 and 663 nm (ε 195 270, 219 498, 14 052, 18 886, 28 588, 86 733).

### Example 3

### Production of "Chlorin NT2H2"

A solution was prepared by dissolving 100 mg Purpurin NT2 in 20 mg tetrahydrofuran and adding 2 drops of triethylamine; with stirring, an addition of 20 mg palladium on charcoal was made and the mixture which resulted was hydrogenated at room temperature of about 22° for 5 hours in a sloping manifold hydrogenator in which a slight positive pressure of hydrogen was maintained. The palladium on charcoal that was used was composed of 10% w/w of palladium and 90% w/w of charcoal. The palladium on charcoal was filtered from the colorless reaction mixture, and the filtrate was stirred vigorously while exposed to air until the solution turned brown, about 2½ hours. The solvent was then removed in vacuo, and the residue was dissolved in the minimum dichloromethane containing 1% v/v of methanol and chromatographed on silica. A major blue band was collected; the solvent was removed; and the crude product was dissolved in 5 ml dichloromethane containing 1% v/v of methanol and recrystallized, yielding 72 mg brown microprisms which were identified by nuclear magnetic resonance as Chlorin NT2H2, a compound having the formula of Fig. 6 of the drawings where R1 to R8 are ethyl, R9 is CO₂CH₂CH₃, and R10 R13 are hydrogen. Chlorin NT2H2 was found to have absorbance peaks in the visible spectrum at 403, 500, 535, 558, 610 and 660 nm (ε 114 650, 23 532, 5 662, 4 246, 8 493, 39 455). The Chlorin NT2H2 zinc complex was prepared by the method described in Example 2; it was found to have absorbance peaks in the visible spectrum at 408, 515, 545, 590 and 633 nm (ε 145 474, 9 858, 5 377, 15 832, 59 444).

The nickel complex of Chlorin NT2H2 was also prepared by the method described in Example 2, except that nickel acetate was substituted for the zinc acetate. The nickel complex of Chlorin NT2H2 was found to have absorbance peaks in the visible spectrum at 405, 498, 533, 588 and 630 nm (ε 145 779, 11 034, 8 693, 19 392, 64 146).

The zinc and nickel complexes have the formula of Fig. 2 of the drawings where R1 to R8 are ethyl, R9 is CO₂CH₂CH₃ and R10 to R13 are hydrogen. M is Zn for the zinc complex and Ni for the nickel complex.

### Example 4

### Production of Purpurin NT2 and Purpurin NT1

A solution of 100 mg meso-(β-ethoxycarbonylvinyl)octaethyl porphyrin in 20 ml glacial acetic acid was heated under reflux in air for 24 hours. The solution was allowed to stand at room temperature of about 22° until it cooled; the solvent was removed in vacuo; and the residue was dissolved in the minimum dichloromethane containing 1% v/v of methanol and chromatographed on silica. First and second major green bands were collected; the solvent was removed from the first band; and the crude product was dissolved in 4 ml dichloromethane containing 1% v/v of methanol and recrystallized, yielding 40 mg "Purpurin NT1", a compound having the formula of Fig. 5 of the drawings where R1 is =CHCH₃, R2 to R8 are ethyl, R9 is CO₂CH₂CH₃ and R10 to R13 are hydrogen. Purpurin NT1 was identified by nuclear magnetic resonance; it has absorbance peaks in the visible spectrum at wavelengths of 438, 510, 540, 583, 653, and 715 nm (ε 104 158, 9 450, 11 130, 15 540, 9 020, 42 629).

The solvent was also removed from the second green band, and the crude product was dissolved in 4 ml dichloromethane containing 1% v/v of methanol and recrystallized, yielding 39 mg Purpurin NT2, which was identified by nuclear magnetic resonance spectroscopy.

Purpurin NT1 was hydrogenated by a procedure similar to that described above in Example 3, yielding, after work-up and chromatographic purification as there described, 65 mg Chlorin NT2H2.

The procedure described in Example 1 has been used to produce other purpurins. Typical ones of the starting materials used and the intermediates and purpurins produced are set forth tabularly in Examples 5, 6, 7 and 8.

### Example 5

| Compound | Formula of |
|---|---|
| Starting material, Nickel meso-formyletio porphyrin I | Fig. 4* |
| First Intermediate, Nickel meso-(β-ethoxycarbonylvinyl)-etio porphyrin I | Fig. 4* |
| Second intermediate, Meso-(β-ethoxycarbonylvinyl)-etio porphyrin I | Fig. 3* |
| "Purpurin ET2" | Fig. 5* |

| | |
|---|---|
| ^{*}Where: R1, R3, R5, and R7 are CH₃, R2, R4, R6, and R8 are CH₂CH₃. In the starting material, R is CHO and M is Ni. In the first intermediate, R is CH=CHCO₂CH₂CH₃ and M is Ni. In the second intermediate, R is CH=CHCO₂CH₂CH₃. In Purpurin ET2, R9 is CO₂CH₂CH₃ and R10 to R13 are hydrogen. | |

The production of nickel meso-formyletio porphyrin I is disclosed in a Journal article by Johnson et al., J.Chem.Soc. (c) 1966, p.794.

### Example 6

| Compound | Formula of |
|---|---|
| Starting Material, Nickel meso-formyl coproporphyrin I tetramethyl ester^{*} | Fig.4^{**} |
| First intermediate, Nickel meso-(β-ethoxycarbonylvinyl)coproporphyrin I tetramethyl ester | Fig.4^{**} |
| Second intermediate, Meso-(β-ethoxycarbonylvinyl)-coproporphyrin I tetramethyl ester | Fig. 3** |
| "Purpurin JP1" | Fig. 5** |

| | |
|---|---|
| ^{*}Produced as subsequently described herein. | |
| ^{**}Where: R1, R3, R5, and R7 are CH₃ and R2, R4, R6 and R8 are CH₂ CH₂ CO₂ CH₃. In the starting material, R is CHO and M is Ni. In the first intermediate, R is CH=CHCO₂CH₂CH₃ and M is Ni. In the second intermediate, R is CH=CHCO₂CH₂CH₃. In Purpurin JP1, R9 is CO₂CH₂CH₃ and R10 to R13 are hydrogen. | |

The nickel meso-formyl coproporphyrin I tetramethyl ester starting material used in the procedure of Example 6 was produced from a commercially available material, coproporphyrin I tetramethyl ester (formula of Fig. 3 of the attached drawings); nickel coproporphyrin I tetramethyl ester was produced therefrom (formula of Fig. 4 where M is Zn). In both cases, R1, R3, R5 and R7 are CH₃ and R2, R4, R6 and R8 are CH₂CH₂CO₂CH₃.

The Ni Coproporphyrin I Tetramethyl ester was prepared from a solution of 100 mg coproporphyrin I tetramethyl ester in a mixed solvent composed of 50 ml dichloromethane and 5 ml methanol and 100 mg nickel acetate. A mixture which was prepared by adding the nickel acetate to the solution was refluxed for about 12 hours until the electronic spectrum of the reaction mixture indicated that chelation was complete. The reaction mixture was then concentrated to 7 ml and allowed to cool to room temperature of about 22°. Product which precipitated was recovered by filtration, dissolved in a mixed solvent composed of 5 ml dichloromethane and 2 ml methanol, and recrystallized, yielding 98 mg Ni coproporphyrin I tetramethyl ester. The compound showed absorbance peaks in the visible spectrum at 392, 515 and 552 nm; the relative intensities at these peaks were 20.19, 1 and 2.56, respectively.

The Nickel-meso-formyl coproporphyrin I tetramethyl ester was prepared from the following materials: 2.8 ml freshly distilled phosphorus oxychloride, 2 ml dry dimethyl formamide, a solution of 100 mg nickel-coproporphyrin I tetramethyl ester in 75 ml dry 1,2-dichloroethane and 75 ml saturated aqueous sodium acetate. The dimethyl formamide was cooled on an ice bath, and the phosphorus oxychloride was added thereto dropwise. The solution which resulted was allowed to stand at room temperature for 30 minutes, and was then warmed to 50°. The nickelcoproporphyrin I tetramethyl ester solution was then added dropwise, with stirring, to the phosphorus oxychloride solution; the addition was made over a period of 30 minutes. The reaction mixture was maintained at about 50°, with stirring, for an additional 2 hours, during which time a change in color from red to green was observed. The sodium acetate solution was then added to the reaction mixture, and stirring was continued for an additional 2 hours. The organic and the aqueous phases were then separated, and the aqueous phase was extracted with dichloromethane. The organic phase and the dichloromethane extract were then combined, and evaporated to dryness. The solid which remained was recrystallized from a solvent composed of equal parts by volume of dichloromethane and methanol, yielding 86 mg red microcrystals which were identified by nuclear magnetic resonance as nickel-mesoformylcoproporphyrin I tetramethyl ester. Absorbance peaks were found in the visible spectrum at 400, 420, 558 and 645 nm, with relative intensities of 10.10, 8.69, 1.02 and 1, respectively.

### Example 7

| Compound | Formula of |
|---|---|
| Starting Material, Nickel meso-formyloctaethylporphyrin | Fig.4^{*} |
| First intermediate, Nickel meso-(β-methoxycarbonylvinyl) octaethylporphyrin | Fig.4^{*} |
| Second intermediate, Meso-(β-methoxycarbonylvinyl)-octaethylporphyrin | Fig.3^{*} |
| "Purpurin GG2" | Fig.5^{*} |

| | |
|---|---|
| ^{*}Where: R1 through R8 are CH₂CH₃. In the starting material, R is CHO and M is Ni. In the first intermediate, R is CH=CHCO₂CH₃ and M is Ni. In the second intermediate, R is CH=CHCO₂CH₃. In Purpurin GG2, R9 is CO₂CH₃ and R10 to R13 are hydrogen. | |

### Example 8

| Compound | Formula of |
|---|---|
| Starting Material, Copro I | Fig. 3 |
| First intermediate, Nickel Copro I | Fig.4^{*} |
| Second intermediate, Nickel meso-(β-ethoxycarbonylvinyl) Copro I | Fig.4^{*} |
| Third intermediate, Meso-(β-ethoxycarbonylvinyl) Copro I | Fig.3^{*} |
| "Purpurin CC1" | Fig.5^{*} |

| | |
|---|---|
| ^{*}Where: R1, R3, R5 and R7 are CH₃, and R2, R4, R6 and R8 are CH₂CH₂CO₂CH₂. In the starting material, R is CHO. In the first intermediate, R is CHO and M is Ni. In the second intermediate, R is CH=CHCO₂CH₂CH₃ and M is Ni. In the third intermediate, R is CH=CHCO₂CH₂CH₃. In Purpurin CC1, R9 is CO₂CH₃ and R10 to R13 are hydrogen. | |

The procedure of Example 3 has been used to hydrogenate Purpurin ET2 and Purpurin JP1, producing Chlorin ET2H2 and Chlorin JP1H2, respectively, where the isocyclic ring (to which the R9 substitutent is attached) is saturated. The chlorins had the same substitutents as the starting purpurins, but the structure of Fig. 6 instead of that of Fig. 5.

The procedure of Example 4 has been used to produce other zinc and nickel complexes. The purpurin and chlorin starting materials, the zinc or nickel compound used, and the complexes produced are set forth below:

| Starting Purpurin or Chlorin | Zinc or nickel Compound | Complex Produced |
|---|---|---|
| Purpurin ET2 | Zinc acetate | Zn |
| Purpurin ET2 | Nickel acetate | Ni |
| Purpurin GG2 | Zinc acetate | Zn |
| Purpurin GG2 | Nickel acetate | Ni |
| Chlorin ET2H2 | Zinc acetate | Zn |
| Chlorin ET2H2 | Nickel acetate | Ni |
| Chlorin NT2H2 | Silver acetate | Ag |
| Purpurin NT1 | Zinc acetate | Zn |
| Purpurin JP1 | Zinc acetate | Zn |
| Chlorin NT2H2 | Tin [2] chloride | Sn |
| Purpurin ET2 | Tin [2] chloride | Sn |

More visible spectrum peak absorbance data follow.

| Compound | Wavelengths, nm (relative intensities) |
|---|---|
| Purpurin ET2 | 406(16.69), 424(15.26), 502(1.36) 531(1), 566(1.5), 695(3.47) |
| Chlorin ET2H2 | 400(70.16), 498(5.53), 530(1.29), 555(1), 606(1.91), 662(20.82) |
| Zn Chlorin ET2H2 | 401(20.36), 530(1), 568(1.18), 630(3.20) |
| Purpurin ET2 | 434(16.44), 530(1), 576(1.31) 612(1.77), 660(5.0) |
| Ni Purpurin ET2 | 434(5.14),657(1) |
| Ni Chlorin ET2H2 | 404(11.70), 497(1), 622(4.41) |
| Purpurin JP1 | 409(22.41), 504(1.67), 541(1.21) 567(1.08), 647(1), 691(3.79) |
| Chlorin JP1H2 | 401(14.53), 650(1) |
| Purpurin GG2 | 406(12.94), 427(19.18), 500(1), 526(1), 565(1.89), 637(0.76), 695(5.25) |
| Zn Purpurin GG2 | 436(8.33), 616(1), 661(3.43) |
| Ni Purpurin GG2 | 427(4.20), 648(1) |

In vitro and in vivo testing of purpurins and chlorins according to the invention was also carried out. For the in vitro testing, the compounds were dissolved in dimethyl sulfoxide or in a solvent that is commercially available under the trade designation PROTOSOLV®, and diluted with phosphate buffer saline to a concentration of 0.010 mg per ml. The tests were conducted on FANFT®(N-[4-(5-nitro-2furyl)2-thiazolyl] formamide) induced rat bladder tumor cells. Two tests were conducted, uptake and toxicity.

The uptake test involved incubating the FANFT® induced rat bladder tumor cells with a solution of a purpurin or with a solution of a chlorin at a concentration of 0.010 mg per ml for one hour, temperature 37°, followed by removal of the incubation media, three washes of the cells with phosphate buffered saline, and extracting and quantitating of the purpurin or chlorin retained by the cells. The procedure as used in investigating the use of HpD in rat tumor cells is described in detail in a journal article by Garbo et al., Analytical Biochemistry, Vol. 151 (No. 1), pp. 70-81, 1985.

The toxicity test involved the incubation and washing steps of the uptake test, followed by illumination of the cells with red light of a wavelength greater than 590 nanometers. Cell survival was then determined by Trypan Blue exclusion, a technique described in a journal article by Schneck, R., Arch. Path. (Lab. Med.) 35, p. 857, 1943.

The uptake test was positive for Purpurin NT2 and for Chlorin NT2H2. The results of the toxicity test are given in the following table, together with the results of toxicity testing of HpD, of phosphate buffer saline and of the solvent system in which the purpurin or chlorin was dissolved.

| Test Solution | Average Viability |
|---|---|
| Purpurin NT2 | 46 |
| Chlorin NT2H2 | 51 |
| HpD | 42 |
| Phosphate buffer saline | 93 |
| Mixed solvent | 96 |

The in vivo testing was conducted on male Fisher 344 rate weighing 135 to 150 g in whom the transplantable FANFT® (N-[4-(5-nitro-2-furyl)-2-thiazolyl]formamide tumor system had been implanted. (Use of this system is reported by Selman, S.H., et al., Cancer Research, pp. 1924-1927, May, 1984.) Two tumors were implanted into the subcutaneous tissue of the abdominal wall of each test animal; when the testing was carried out, each tumor was about 1 cm in diameter.

The purpurins and chlorins tested were dissolved in a commercially available non-ionic solubilizer and emulsifier obtained by reacting ethylene oxide with castor oil in a ratio of 35 moles of ethylene oxide per mole of castor oil, diluting the resulting solution with 1,2-propanediol, and producing an emulsion with the resulting solution and 0.9% w/w aqueous sodium chloride solution. The specific non-ionic solubilizer used is available from BASF under the designation CREMOPHOR EL®; it is composed of fatty acid esters of polyglycols, glycerol polyglycols, polyethylene glycols and ethoxylated glycerol. The test solutions were prepared from 50 mg purpurin or chlorin, 1 or 2 ml warm solubilizer (enough to dissolve the test compound), enough 1,2-propanediol to make a solution of the purpurin or chlorin in a mixed diol/solubilizer solvent containing 32.9% w/w solubilizer; finally, enough 0.9% w/w aqueous sodium chloride was added to make 10 ml test solution so that the final concentration of the purpurin or chlorin in the test solution was 5 mg per ml. Each test solution was made, with mechanical shaking and stirring, by dissolving the purpurin or chlorin in the solubilizer, diluting the resulting solution with the indicated amount of 1,2-propanediol, and adding the sodium chloride solution to the diluted solution. A control solution was also prepared for use with each test solution. The control was identical with the test solution except that it contained no purpurin or chlorin. The test solutions were prepared in air, but it is believed that a nitrogen atmosphere would be advantageous because it would minimize the chance of a reaction with oxygen.

The testing involved injecting each rat with a solution of the purpurin or chlorin under test, dosage 4 mg purpurin or chlorin per kg of body weight or 10 mg purpurin or chlorin per kg of body weight or with the same volume of the appropriate control, irradiating one of the two tumors with light for 30 minutes, sacrificing the animals, and examining the tumors. The injections were made via the dorsal tail vein. The irradiation of one of the tumors occurred twenty four hours after each rat was injected while the other of the two tumors was shielded by an opaque box.

Tumor temperature and body core temperature were monitored, using thermistors, one placed into the tumor and one placed intrarectally. Tumor temperature was kept within 2°C of body core temperature by directing a jet of cool air over the tumor.

The light source was a slide projector that had a 500 watt bulb fitted with a red filter which is available from Corning Glass Works under the designation 2418. The light was reflected 90°C by a silvered mirror, and was focused onto the tumor with a secondary condensing lens. The light intensity on the tumor was monitored, using a photometer /radiometer that is available from United Detector Technology under the designation "UDT #351", and was maintained at 200 mw per cm².

Six rats were injected with the purpurin or chlorin test solution and two were injected with the appropriate control solution.

Four hours after the irradiation, three of the rats that had been injected with the test solution and one of the rats that had been injected with the control were sacrificed by an intracardiac injection of saturated aqueous potassium chloride solution. Twenty four hours after the irradiation, another three of the rats that had been injected with the test solution and the other rat that had been injected with the control were sacrificed in the same way. During the testing, the rats were under barbituate anesthesia (65 mg per kg body weight).

The tumors were then excised, placed in 10% w/w phosphate-buffered formalin and cut into three sections perpendicular to their long axis. The tumors were then embedded in paraffin and cut into sections five microns in width. The sections were stained with hematoxylin and eosin.

Histologic examination of the stained sections revealed approximately comparable areas of hemorrhage and tumor cell necrosis in specimens removed four hours after irradiation from animals that had been injected with Purpurin NT2, with Purpurin GG2, and with Purpurin ET2. However, tumor cells which appeared to be viable were observed. Tumor necrosis was extensive in specimens removed twenty four hours after irradiation from animals that had been injected with Purpurin NT2, with Purpurin GG2, and with Purpurin ET2; no viable tumor cell was observed in these specimens. No change in the tumors was observed in the specimens that were removed from animals that had been injected with the control solution. Tumor necrosis was complete in specimens removed from animals that had been injected with purpurin NT1 both four hours after irradiation and twenty four hours after irradiation. However, the irradiation was found to have caused extensive liver damage to some of the animals. The liver damage is believed to have occurred because of the high absorbance peak of Purpurin NT1 at 715 nm and the relative transparency of tissue to light of such wavelength. There was residual Purpurin NT1 in the liver which caused the damage when irradiated. This means, however, that Purpurin NT1 is highly effective when properly used.

The in vivo test procedure described above has also been used to evaluate solutions in which the Purpurin NT2, Purpurin GG2, Purpurin NT1 and Purpurin ET2 were replaced by Chlorin NT2H2 and by Chlorin ET2H2. Histologic examination of the stained sections from rats into which the Chlorin NT2H2 and ET2H2 solutions had been injected indicated that these chlorins were substantially equivalent in this test and were similar to Purpurin NT2, to Purpurin GG2 and to Purpurin ET2, the only difference observed being that hemorrhage within the tumors was less pronounced with the chlorins.

Purpurin JP1, zinc Purpurin ET2, tin Chlorin NT2H2, Tin Purpurin ET2, silver Chlorin NT2H2, zinc Purpurin NT1 and zinc Purpurin JP1 were all found, by the above-described in vivo test procedure, to cause tumor necrosis.

Several compounds were selected for testing by the foregoing in vivo test procedure, but at lower dosages. Results of some of the testing of zinc Purpurin ET2 ("ZnET2"), tin Chlorin NT2H2 ("SnNT2H2"), tin Purpurin ET2 ("SnET2"), Purpurin ET2 ("ET2"), Purpurin NT2 ("NT2") and Purpurin NT1 ("NT1") are summarized in the following table:

| Compound Tested | Dosage, mg/kg | Results |
|---|---|---|
| ZnET2 | 0.25 | The tumors on all of the the test animals were smaller twelve days after than they were before irradiation |
| SnNT2H2 | 0.25 | 80% of the test animals were free of tumors twelve days after irradiation. |
| SnET2 | 0.25 | 40% of the test animals were free of tumors twelve days after irradiation. |
| ET2 | 1.0 | 50% of the test animals were free of tumors twelve days after irradiation. |
| NT2 | 1.0 | The tumors on all of the test animals were smaller twelve days after than they were before irradiation. |
| NT1 | 1.0 | 20% of the test animals were free of tumors twelve days after irradiation. |

The production of Purpurin NT2, of Purpurin ET2, of Purpurin JP1, of Purpurin GG2, and of Purpurin CC1 is described in Examples 1, 5, 6, 7 and 8 hereof, respectively. In each case, the purpurin had the structure of Fig. 5 of the attached drawings where R1 through R8 had certain meanings and R10 to R13 were hydrogen; the purpurins were produced from metal complexes of porphyrins having the structure of Fig. 4 of the attached drawings where R1 through R8 meant the same as in the purpurins, and R was CH=CHCO₂CH₂CH₃ or CH=CHCO₂CH₃. Because of the identity of R in the porphyrin starting materials, R9 was CO₂CH₂CH₃ or CO₂CH₃ in the foregoing purpurins. The other porphyrins which are required for substitution in the procedure of Example 1 for nickel meso-formyl octaethyl porphyrin to produce the purpurins having the structure of Fig. 5 of the drawings where R10 to R13 are hydrogen are either disclosed in the literature or can be produced by methods that are disclosed in the literature. In general, porphyrins are produced by condensing two pyrroles, and by then condensing two of the condensation products. The two condensation products can be the same or different, and each can be made by condensing two pyrroles that are the same or different. The pyrroles necessary to produce porphyrins having the structure of Fig. 3 of the attached drawings (without the alkoxycarbonylvinyl substituent, R) where each of R1 to R8 is
H or CHO,
a primary or secondary alkyl group having from 1 to 4 carbon atoms,
an alkylene group having from 2 to 4 carbon atoms,
a group having the formula R₂N(R₃)₂ where R₂ is a bivalent aliphatic hydrocarbon radical having from 1 to 4 carbon atoms, wherein any carbon to carbon bond is either a single or a double bond, and not more than one is a double bond; R₃ is hydrogen or an alkyl radical having from 1 to 2 carbon atoms and the two R₃ groups can be the same or different,
a group having the formula R₂N(R₄)₃⁺ where R₂ is a bivalent aliphatic hydrocarbon radical having from 1 to 4 carbon atoms, wherein any carbon to carbon bond is either a single or a double bond, and not more than one is a double bond; and R₄ is an alkyl group having from 1 to 2 carbon atoms and the three R₄ groups can be the same or different,
a group having the formula R₂OH were R₂ is a bivalent aliphatic hydrocarbon radical having from 1 to 4 carbon atoms, wherein any carbon to carbon bond is either a single or a double bond, and not more than one is a double bond, or
CO₂R', CH₂CO₂R' or CH₂CH₂CO₂R' where R' is H, or a primary or secondary alkyl group having from one to four carbon atoms,
are all known or can be made by known methods. The alkoxycarbonylvinyl group can then be introduced in the known way disclosed in Example 1 hereof. These porphyrins produce, when used in the procedure of Example 1, purpurins having the structure of Fig. 5 of the attached drawings where R10 to R13 are hydrogen. Such purpurins can be reacted with the Vilsmier reagent to introduce a formyl group as R10 and R13, or chlorins can be produced therefrom by the method of Example 3 and the Vilsmier reagent can be used to introduce a formyl group as R10 in the chlorin. The formyl group, after separation of the isomers, if necessary, can be reduced to CH₃, or can be reduced to CH₂OH or converted to an oxime group, which can then be converted to a cyano group, which, in turn, can be converted to an amide. The formyl group can also be reacted with Wittig reagents to give alkyl, alkenyl or carboxy side chains or to introduce the previously identified substituents which have an amine or an alcoholic OH function in the R10 or in the R13 position. After the desired group has been introduced as R10, R13, or both, the purpurin or chlorin can be reacted in the same way to introduce a desired group as R11. Finally, the chemistry can be used to introduce a desired group as R12. The corresponding chlorins can be produced from the purpurins by the method described in Example 3; the corresponding purpurins can be produced from the chlorins by oxidation; and the metal complexes can be produced by the method of Example 2 or by modifications thereof which are subsequently discussed herein. The method of Example 4, i.e., cyclizing the porphyrin in air, can be used to produce other purpurins having an R1 substituent connected thereto by a carbon to carbon double bond; specifically, the method can be used to produce purpurins where R1 is a bivalent aliphatic hydrocarbon radical having from 2 to 4 carbon atoms wherein both of the valences of the radical are attached to the same carbon atom thereof and to a carbon atom of the purpurin, chlorin, or metal complex. Hydrogenation to convert these purpurins to chlorins, however, also saturates the R1 double bond. It will be appreciated that the R1 double bond forms in the Example 4 procedure because a hydroxyl group is introduced into the molecule and, at the temperature of reflux, the elements of water are eliminated to form the double bond. Reaction of the hydroxyl group with hydrogen can be prevented by cyclizing at a lower temperature; the resulting purpurin can then be hydrogenated to the corresponding chlorin and the double bond with R1 can be formed by heating. It will be appreciated, however, that purpurins which are connected to the R1 substituent through a double bond are preferred over the corresponding chlorins because of the greater ease of synthesis.

It will also be appreciated that purpurins and chlorins according to the invention where R10 through R13 are hydrogen are preferred, other factors being equal, because the production of the compounds with other groups in these positions is complicated, time consuming and expensive. Purpurins and chlorins according to the invention where R9 is CO₂R' and R' is a primary or secondary alkyl group having from 1 to 4 carbon atoms are also preferred, other factors being equal, because these groups are present at the end of the ring closure reaction which produces the purpurins (see Example 1). However, the esters of these R9 sustituents can be reduced to formyl groups and reacted as discussed above to introduce any of the other R1 to R8 or R10 to R13 substituents.

The method of Example 2, supra, can be used to produce metal complexes of other purpurins and of various chlorins. Specifically, an equivalent amount of another purpurin or of a chlorin can be substituted for the Purpurin NT2, or copper acetate, nickel acetate, cobalt acetate, silver acetate, palladium acetate, or platinum acetate can be substituted for the zinc acetate, or both substitutions can be made. In this manner, purpurin metal complexes having the formula of Fig. 1 where M is one of the metals named above in this paragraph can be produced from purpurins having the formula of Fig. 5; chlorin metal complexes having the formula of Fig. 2 where M has the same meaning can be produced from chlorins having the formula of Fig. 6. Other complexes can be produced by the method of Example 2 from salts containing cations other than acetate, and producing complexes which have the structures or Figs. 2 and 5, but where M does not represent merely a metal anion. Examples of salts that can be substituted for zinc acetate in the Example 2 procedure are identified below, together with the identity of M in Figs. 2 and 5:

| Salt | Identity of M |
|---|---|
| FeCl₃ | Fe(Cl) |
| MnCl₄ | Mn(Cl) |
| InCl₃ | In(Cl) |
| VCl₄* | V(O) |
| Tl(CF₃CO₂)₃ | Tl(OAc)(H₂O) |
| SnCl₂ | Sn(OH)₂ |
| [Rh(CO)₂Cl]₂ | Rh(Cl)(H₂O) |

| | |
|---|---|
| ^{*}Using phenol as the solvent instead of glacial acetic acid. | |

The procedure of Example 2 can also be modified by substituting phenol for glacial acetic acid and metal chelates of pentane, 2,4-dione for zinc acetate to produce complexes of any of the purpurins and chlorins. Metals that can be so reacted (as pentane, 2,4-dione chelates) and the identity of M in the complex that is produced are set forth in the following table:

| Metal | Identity of M | Metal | Identity of M |
|---|---|---|---|
| Al | Al(acac)^{*} | Th | Th(acac)₂ |
| Sc | Sc(acac) | U | U(acac)₂ |
| Ga | Ga(acac) | La | La(acac₂ |
| In | In(acac) | Ce | Ce(acac) |
| Mo | Mo(acac) | Nd | Nd(acac) |
| Ti | Ti(acac)₂ | Sm | Sm(acac) |
| Zr | Zr(acac)₂ | Gd | Gd(acac) |
| Hf | Hf(acac)₂ | Tb | Tb(acac) |
| Eu | Eu(acac) | Dy | Dy(acac) |
| Pr | Pr(acac) | Ho | Ho(acac) |
| Yb | Yb(acac) | Er | Er(acac) |
| Y | Y(acac) | Tm | Tm(acac) |
| Lu | Lu(acac) | | |

| | | | |
|---|---|---|---|
| ^{*}The pentane, 2,4-dione portion of a chelate thereof with a metal. | | | |

Complexes of any of the foregoing purpurins and chlorins can also be produced by the procedure of Example 2, substituting dimethylformamide for glacial acetic acid and CrCl₂ for zinc acetate. Metal complex formation occurs at higher temperatures when dimethylformamide is used, because of its higher boiling temperature. M in the complexes is Cr(OH).

Similarly, complexes of the foregoing purpurins and chlorins can be produced by the procedure of Example 2, substituting pyridine for glacial acetic acid and PbCl₂ for zinc acetate. M in the complexes is Pb.

The production of purpurin solutions in the specific non-ionic solubilizer that is available under the designation CREMOPHOR EL®, and the production of emulsions of such solutions with 1,2-propanediol and saline solution is described above, as is the use of such solutions to detect and treat tumors. It will be appreciated that purpurins, chlorins and their metal complexes can be dissolved in other non-ionic solubilizers and that the solutions can be used to produce emulsions that can be administrated intravenously. For example, other reaction products of ethylene oxide and castor oil can be so used, as can reaction products of ethylene, propylene and other similar oxides with other fatty acids and the reaction products of propylene and other similar oxides with castor oil. Similarly, glycols other than 1,2-propanediol can be used in producing the emulsions for intravenous administration, or the glycol can be omitted, particularly if the solubilizer is prepared to have a lower viscosity and greater compatibility with water, by comparison with the solubilizer that is available under the designation CREMOPHOR EL. It is necessary only that the solution or emulsion be one which is physiologically acceptable and of a suitable concentration, or dilutable to a suitable concentration, for intravenous administration or for local administration, should that be desirable. An indefinitely large number of such solutions and emulsions will be apparent to those skilled in the relevant art from the foregoing specific disclosure. Similarly, the aqueous phase need not be 0.9% w/w or any other concentration of sodium chloride. Such saline is presently favored for intravenous administration, but other aqueous phases can also be used, so long as the entire composition is physiologically acceptable for intravenous administration and, in fact, other aqueous phases may subsequently be favored. Indeed, other aqueous phases or organic phases may also be favored for local administration.

Dosages ranging from 0.25 to 10 mg per kg of body weight were used in the in vivo procedures described above. It has been determined only that the biological consequences described above were caused by the dosages administered, not that any dosage reported is either a minimum or a maximum. It will be appreciated, therefore, that it is necessary only to use an effective amount of a purpurin or chlorin according to the invention in the detection and treatment of tumors, preferably as small a dosage as possible, and that the exact dosage can be determined by routine experimentation. While systemic administration has been described above, specifically intravenous, it will also be appreciated that local administration will be suitable, at least in some instances.

Illumination of tumors containing a purpurin, a chlorin or a metal complex in accordance with the instant invention can be a surface illumination with a conventional light source, as described above, or can be a surface illumination with a laser. The illumination can also be into the the body or a tumor, for example through optical fibers inserted thereinto.

## Claims

1. A solution in an organic solvent, of a solute that is a purpurin having the structure of Fig. 5, below, a metal complex of a purpurin having the structure of Fig. 1, below, a chlorin having the structure of Fig. 6, below, or a metal complex of a chlorin having the structure of Fig. 2, below: 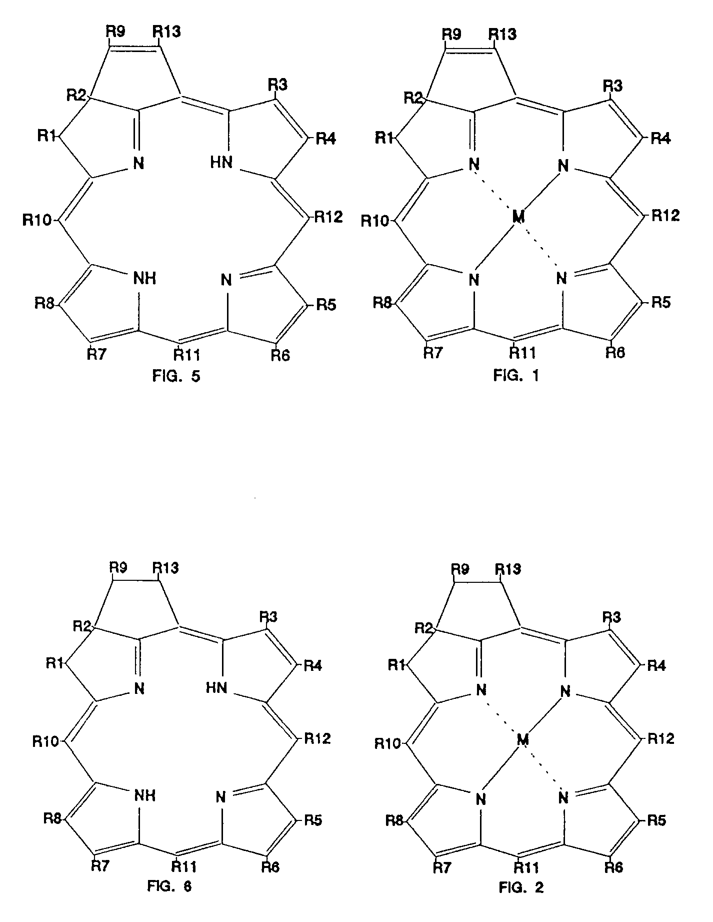 wherein M is Ag, Al, Ce, Co, Cu, Cr, Dy, Er, Eu, Fe, Ga, Gd, Hf, Ho, In, La, Lu, Mn, Mo, Nd, Pb, Pd, Pr, Pt, Rh, Sb, Sc, Sm, Sn, Tb, Th, Ti, Tl, Tm, U, V, Y, Yb, Zn or Zr, and each of R1 to R13 is:
H or CHO,
an alkyl group other than t-butyl having from 1 to 4 carbon atoms,
an alkylene group having from 2 to 4 carbon atoms,
a bivalent aliphatic hydrocarbon radical having from 2 to 4 carbon atoms wherein both of the valences of the radical are attached to the same carbon atom thereof and to a carbon atom of the purpurin, chlorin, or metal complex,
a group having the formula R₂N(R₃)₂ where R₂ is a bivalent aliphatic hydrocarbon radical having from 1 to 4 carbon atoms, wherein any carbon to carbon bond is either a single or a double bond, and not more than one is a double bond; R₃ is hydrogen or an alkyl group having from 1 to 2 carbon atoms and the two R₃ groups can be the same or different,
a group having the formula R₂N(R₄)₃A where R₂ is a bivalent aliphatic hydrocarbon radical having from 1 to 4 carbon atoms, wherein any carbon to carbon bond is either a single or a double bond, and not more than one is a double bond; A is a physiologically acceptable anion; and R₄ is an alkyl group having from 1 to 2 carbon atoms and the three R₄ groups can be the same or different,
a group having the formula R₂OH were R₂ is a bivalent aliphatic hydrocarbon radical having from 1 to 4 carbon atoms, wherein any carbon to carbon bond is either a single or a double bond, and not more than one is a double bond, or
CO₂R', CH₂CO₂R' or CH₂CH₂CO₂R', where R' is hydrogen or an alkyl group other than t-butyl having from 1 to 4 carbon atoms,
with the proviso that not more than one of R1 through R13 is CHO, a group having the formula R₂N(R₃)₂, or a group having the formula R₂N(R₄)₃A, and with the further proviso that only R1 can be a bivalent hydrocarbon radical having from 2 to 4 carbon atoms wherein both of the valences of the radical are attached to the same carbon atom thereof and to a carbon atom of the purpurin, chlorin, or metal complex, and wherein the solvent comprises an organic liquid, and is one which is physiologically acceptable and of a suitable concentration or dilutable to a suitable concentration for intravenous administration, for use as an active pharmaceutical substance.

2. A solution in a solvent according, to claim 1 as a substance for use in the detection and treatment of tumours.

3. An aqueous emulsion or suspension of a solution as claimed in claim 1 of a purpurin having the structure of Fig. 5.

4. An aqueous emulsion or suspension of a solution as claimed in claim 1 of a metal complex of a purpurin having the structure of Fig. 1.

5. An aqueous emulsion or suspension of a solution as claimed in claim 1 of a chlorin having the structure of Fig. 6.

6. An aqueous emulsion or suspension of a solution as claimed in claim 1 of a metal complex of a chlorin having the structure of Fig. 2.

7. A purpurin or a chlorin having the structure of Fig. 5 or 6, below, or a metal complex of a purpurin or a chlorin having the structure of Fig. 1 or 2, below, 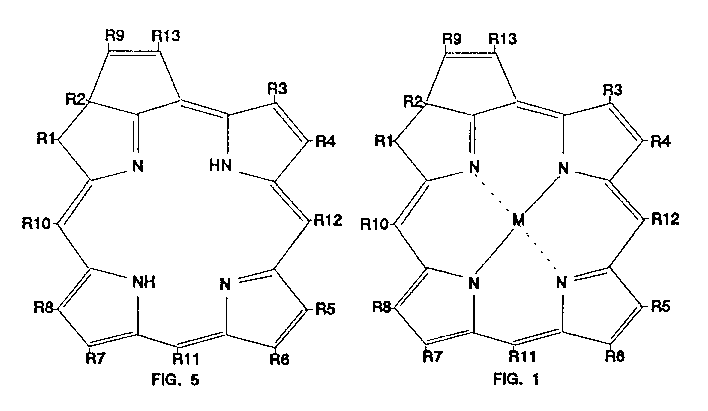 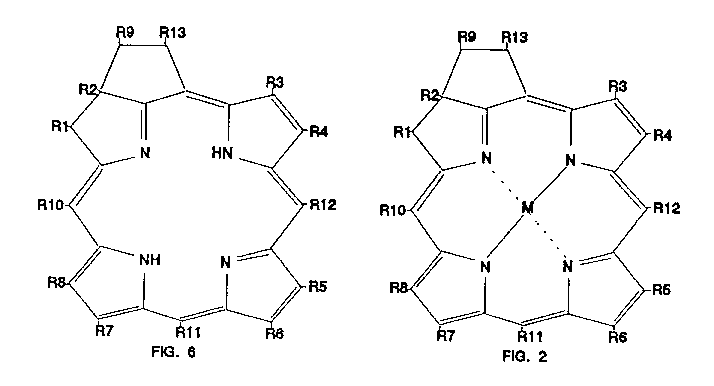 wherein M is Ag, Al, Ce, Co, Cr, Cu, Dy, Er, Eu, Fe, Ga, Gd, Hf, Ho, In, La, Lu, Mn, Mo, Nd, Pb, Pd, Pr, Pt, Rh, Sb, Sc, Sm, Sn, Tb, Th, Ti, Tl, Tm, U, V, Y, Yb, Zn or Zr, and each of R1 to R13 is
H or CHO,
an alkyl group other than t-butyl having from 1 to 4 carbon atoms,
an alkylene group having from 2 to 4 carbon atoms,
a bivalent aliphatic hydrocarbon radical having from 2 to 4 carbon atoms wherein both of the valences of the radical are attached to the same carbon atom thereof and to a carbon atom of the purpurin, chlorin, or metal complex,
a group having the formula R₂N(R₃)₂ where R₂ is a bivalent aliphatic hydrocarbon radical having from 1 to 4
carbon atoms, wherein any carbon to carbon bond is either a single or a double bond, and not more than one is a double bond; R₃ is hydrogen or an alkyl group having from 1 to 2 carbon atoms and the two R₃ groups can be the same or different,
a group having the formula R₂N(R₄)₃A where R₂ is a bivalent aliphatic hydrocarbon radical having from 1 to 4 carbon atoms, wherein any carbon to carbon bond is either a single or a double bond, and not more than one is a double bond; A is a physiologically acceptable anion; and R₄ is an alkyl group having from 1 to 2 carbon atoms and the three R₄ groups can be the same or different,
a group having the formula R₂OH were R₂ is a bivalent aliphatic hydrocarbon radical having from 1 to 4 carbon atoms, wherein any carbon to carbon bond is either a single or a double bond, and not more than one is a double bond, or
CO₂R', CH₂CO₂R' or CH₂CH₂CO₂R', where R¹ is hydrogen or an alkyl group other than t-butyl having from 1 to 4 carbon atoms,
with the proviso that not more than one of R1 through R13 is CHO, a group having the formula R₂N(R₃)₂, or a group having the formula R₂N(R₄)₃A, with the further proviso that only R1 can be a bivalent hydrocarbon radical having from 2 to 4 carbon atoms wherein both of the valences of the radical are attached to the same carbom atom thereof and to a carbon atom of the purpurin, chlorin, or metal complex, and with the final proviso that four purpurins having the structure of Fig. 5 are excluded from the claim, namely, two where R1 through R8 are ethyl, R9 is either CHO or CO₂CH₃, and R10 through R13 are H, and two where R1, R4, R6 and R8 are CH₃, R2 is CH₂CH₂CO₂CH₃, R3 and R9 are CO₂CH₃, R5 is CH₂CH₃, R7 is either -CH=CH₂ or -CH2CH₂NHAc, and R10 through R13 are H.

8. A purpurin, a chlorin, a purpurin metal complex or a chlorin metal complex as claimed in claim 6 wherein each of R10 to R13 is hydrogen.

9. A purpurin or a metal complex of a purpurin as claimed in claim 7.

10. A chlorin or a metal complex of a chlorin as claimed in claim 7.

11. A purpurin as claimed in claim 8 where each of R1, R3, R5 and R7 is methyl, each of R2, R4, R6 and R8 is ethyl, and R9 is CH₃CH₂O₂C-.

12. A chlorin as claimed in claim 8 where each of R1, R3, R5 and R7 is methyl, each of R2, R4, R6 and R8 is ethyl, and R9 is CH₃CH₂O₂C-.

13. A purpurin metal complex as claimed in claim 8 where each of R1, R3, R5 and R7 is methyl, each of R2, R4, R6 and R8 is ethyl, and R9 is CH₃CH₂O₂C-.

14. A chlorin metal complex as claimed in claim 8 where each of R1, R3, R5 and R7 is methyl, each of R2, R4, R6 and R8 is ethyl, and R9 is CH₃CH₂O₂C-.

15. A chlorin, a chlorin metal complex or a purpurin metal complex as claimed in claim 7, wherein each of R1 to R8 is an alkyl group other than t-butyl having from 1 to 4 carbon atoms, R9 is CO₂R', CH₂CO₂R' or CH₂CH₂CO₂R', where R' is hydrogen or an alkyl group other than t-butyl having from 1 to 4 carbon atoms, and M is Sn or Zn.

16. A purpurin as claimed in claim 7 wherein, each of R1 to R8 is an alkyl group other than t-butyl having from 1 to 4 carbon atoms, and R9 is CO₂R', CH₂CO₂R' or CH₂CH₂CO₂R', where R' is hydrogen or an alkyl group other than t-butyl having from 2 to 4 carbon atoms.

17. A purpurin metal complex as claimed in claim 12 wherein M is Sn or Zn.

18. A chlorin metal complex as claimed in claim 13 wherein M is Sn or Zn.

## Patentansprüche

1. Eine Lösung, bestehend aus einem organischen Lösungsmittel und einer gelösten Substanz, die ein Purpurin mit der Strukturformel der Abb. 5, ein Metallkomplex eines Purpurins mit der Strukturformel der Abb. 1, ein Chlorin mit der Strukturformel der Abb. 6 oder ein Metallkomplex eines Chlorins mit der Strukturformel der Abb. 2 sein kann (Abb. 1, 2, 5 und 6 siehe unten):
(Anm.d.Ü: Es folgen die Abbildungen 5, 1, 6, 2 der Vorlage) 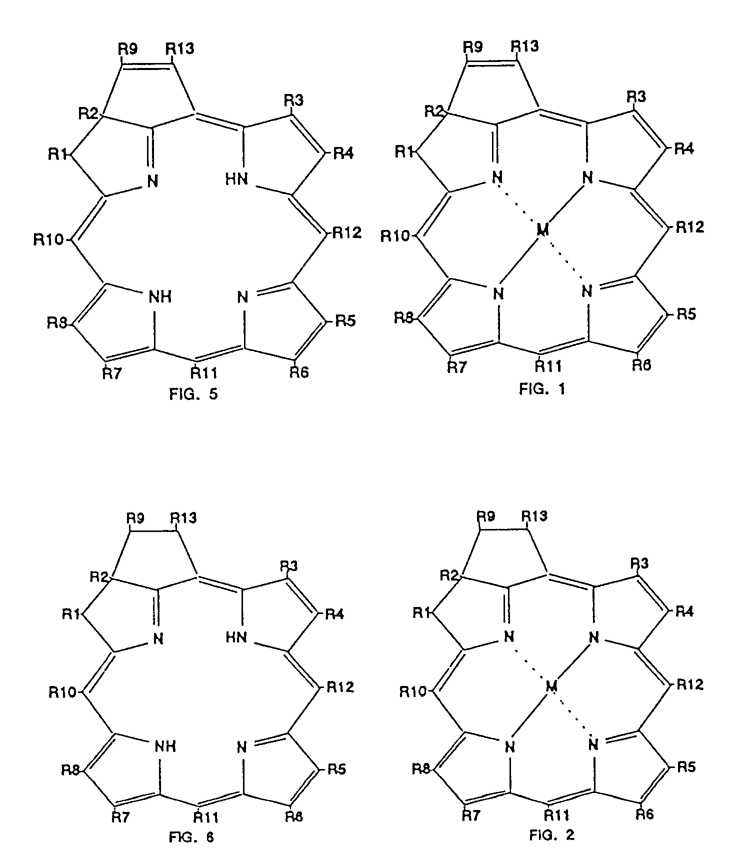 Darin steht M für: Ag, Al, Ce, Co, Cu, Cr, Dy, Er, Eu, Fe, Ga, Gd, Hf, Ho, In, La, Lu, Mn, Mo, Nd, Pb, Pd, Pr, Pt, Rh, Sb, Sc, Sm, Sn, Tb, Th, Ti, Tl, Tm, U, V, Y, Yb, Zn oder Zr und jeder der R1 bis R13 steht für:
H oder CHO,
einen Alkylrest mit 1 bis 4 C-Atomen, mit Ausnahme des t-Butylrestes,
einen Alkylenrest mit 2 bis 4 C-Atomen,
einen zweiwertigen aliphatischen Kohlenwasserstoffrest mit 2 bis 4 C-Atomen,
dessen beide Valenzen, von einem seiner C-Atome ausgehend, mit einem C-Atom des Purpurins, Chlorins oder von deren Metallkomplexen verbunden sind,
einen Rest mit der Formel R₂N(R₃)₂, in der R₂ für einen zweiwertigen aliphatischen Rest mit 1 bis 4 C-Atomen steht, dessen C-C-Bindungen Einfach- oder Doppelbindungen sind und der nicht mehr als eine Doppelbindung enthält und R₃ für ein H-Atom oder einen Alkylrest mit 1 bis 2 C-Atomen steht, wobei die beiden R₃-Reste identisch oder verschieden sein können,
einen Rest mit der Formel R₂N(R₄)₃A, in dem R₂ für einen zweiwertigen aliphatischen Rest mit 1 bis 4 C-Atomen steht, dessen C-C-Bindungen Einfach- oder Doppelbindungen sind und der nicht mehr als eine Doppelbindung enthält, A für ein physiologisch verträgliches Anion, R₄ für einen Alkylrest mit 1 bis 2 C-Atomen steht und dessen drei R₄-Reste identisch oder verschieden sein können,
einen Rest mit der Formel R₂OH, in dem R₂ für einen zweiwertigen aliphatischen Kohlenwasserstoffrest steht, der 1 bis 4 C-Atome enthält, dessen C-C-Bindungen Einfach- oder Doppelbindungen sind und der nicht mehr als eine Doppelbindung enthält oder
CO₂R', CH₂CO₂R' oder CH₂CH₂CO₂R', wobei R' für ein H-Atom oder einen Alkylrest mit 1 bis 4 C-Atomen steht, mit Ausnahme des t-Butylrestes, mit der Bedingung, daß höchstens einer der R1 bis R13 ein CHO-Rest, ein Rest mit der Formel R₂N(R₃)₂ oder ein Rest mit der Formel R₂N(R₄)₃A und mit der weiteren Bedingung, daß nur R1 ein zweiwertiger Kohlenwasserstoffrest mit 2 bis 4 C-Atomen sein kann, dessen beide Valenzen, von einem seiner C-Atome ausgehend, mit einem C-Atom des Purpurins, Chlorins oder von deren Metallkomplexen verbunden sind, wobei das Lösungsmittel ein organisches Lösungsmittel ist, die physiologisch verträglich sind, zur intravenösen Anwendung durch Verdünnen auf die physiologische Konzentration einstellbar sind oder bereits in dieser Konzentration vorliegen, um als pharmakologisch wirksame Substanz eingesetzt werden zu können.

2. Eine Lösung in einem Lösungsmittel, wie unter 1 beansprucht, als Mittel zum Nachweis und zur Behandlung von Tumoren.

3. Eine wässrige Emulsion oder Suspension einer Lösung eines Purpurins wie unter Anspruch 1 beschrieben, mit der Strukturformel der Abb. 5.

4. Eine wässrige Emulsion oder Suspension einer Lösung eines Metallkomplexes eines Purpurins wie unter Anspruch 1 beschrieben, mit der Strukturformel der Abb. 1.

5. Eine wässrige Emulsion oder Suspension einer Lösung eines Chlorins wie unter Anspruch 1 beschrieben, mit der Strukturformel der Abb. 6.

6. Eine wässrige Emulsion oder Suspension einer Lösung eines Metallkomplexes eines Chlorins wie unter Anspruch 1 beschrieben, mit der Strukturformel der Abb. 2.

7. Ein Purpurin oder ein Chlorin mit der Strukturformel der Abb. 5 oder 6 oder ein Metallkomplex eines Purpurins oder eines Chlorins mit der Struktur der Abb. 1 oder 2 (Abb. 1, 2, 5 und 6 siehe unten)
(Anm.d.Ü: Es folgen die Abbildungen 5, 1, 6, 2 der Vorlage) 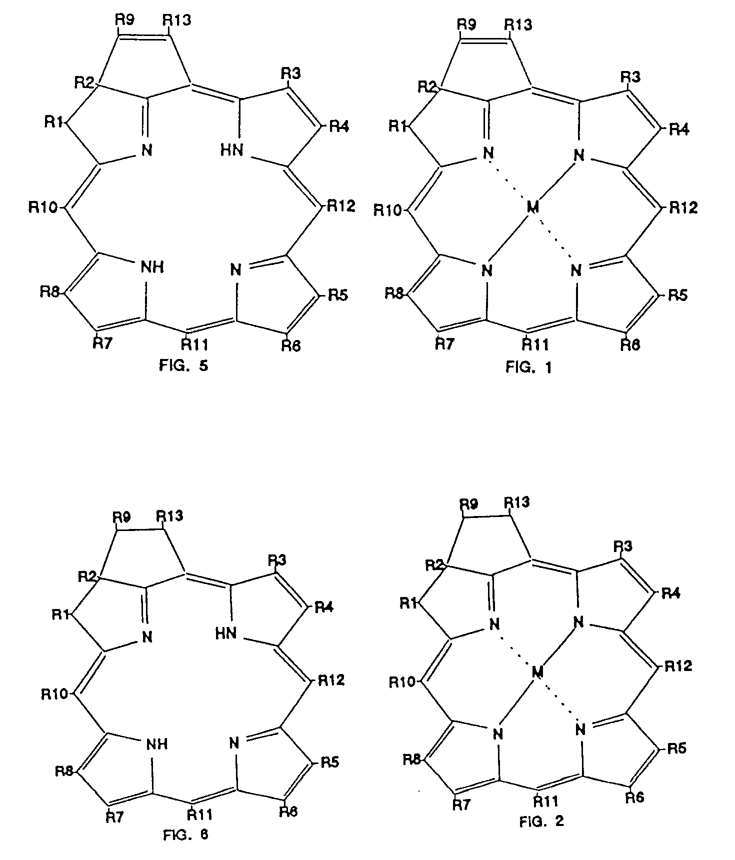 Darin steht M für: Ag, Al, Ce, Co, Cr, Cu, Dy, Er, Eu, Fe, Ga, Gd, Hf, Ho, In, La, Lu, Mn, Mo, Nd, Pb, Pd, Pr, Pt, Rh, Sb, Sc, Sm, Sn, Tb, Th, Ti, Tl, Tm, U, V, Y, Yb, Zn oder Zr und jeder der R1 bis R13 steht für:
H oder CHO,
einen Alkylrest mit 1 bis 4 C-Atomen, mit Ausnahme des t-Butylrestes,
einen Alkylenrest mit 2 bis 4 C-Atomen,
einen zweiwertigen aliphatischen Kohlenwasserstoffrest mit 2 bis 4 C-Atomen, dessen beide Valenzen, von einem seiner C-Atome ausgehend, mit einem C-Atom des Purpurins, Chlorins oder von deren Metallkomplexen verbunden sind,
einen Rest mit der Formel R₂N(R₃)₂, in der R₂ für einen zweiwertigen aliphatischen Rest mit 1 bis 4 C-Atomen steht, dessen C-C-Bindungen Einfach- oder Doppelbindungen sind und der nicht mehr als eine Doppelbindung enthält und R₃ für ein H-Atom oder einen Alkylrest mit 1 bis 2 C-Atomen steht, wobei die beiden R₃-Reste identisch oder verschieden sein können,
einen Rest mit der Formel R₂N(R₄)₃A, in dem R₂ für einen zweiwertigen aliphatischen Rest mit 1 bis 4 C-Atomen steht, dessen C-C-Bindungen Einfach- oder Doppelbindungen sind und der nicht mehr als eine Doppelbindung enthält, A für ein physiologisch verträgliches Anion, R₄ für einen Alkylrest mit 1 bis 2 C-Atomen steht und dessen drei R₄ -Reste identisch oder verschieden sein können,
einen Rest mit der Formel R₂OH, in dem R₂ für einen zweiwertigen aliphatischen Kohlenwasserstoffrest steht, der 1 bis 4 C-Atome enthält, dessen C-C-Bindungen Einfach- oder Doppelbindungen sind und der nicht mehr als eine Doppelbindung enthält oder
CO₂R', CH₂CO₂R' oder CH₂CH₂CO₂R', wobei R' für ein H-Atom oder einen Alkylrest mit 1 bis 4 C-Atomen steht, mit Ausnahme des t-Butylrestes, mit der Bedingung, daß höchstens einer der R1 bis R13 ein CHO-Rest, ein Rest mit der Formel R₂N(R₃)₂ oder ein Rest mit der Formel R₂N(R₄)₃A und mit der weiteren Bedingung, daß nur R1 ein zweiwertiger Kohlenwasserstoffrest mit 2 bis 4 C-Atomen sein kann, dessen beide Valenzen, von einem seiner C-Atome ausgehend, mit einem C-Atom des Purpurins, Chlorins oder deren Metallkomplexen verbunden sind, und mit einer letzten Bedingung, daß vier Purpurine mit der Strukturformel der Abb. 5 von den Ansprüchen ausgeschlossen sind, nämlich zwei, bei denen R1 bis R8 für Ethylreste, R9 für einen CHO- oder einen CO₂CH₃-Rest und R10 bis R13 für H-Atome, und zwei, bei denen R1, R4, R6 und R8 für CH₃, R2 für CH₂CH₂CO₂CH₃, R3 und R9 für CO₂CH₃, R5 für CH₃CH₃, R7 entweder für -CH=CH₃ oder für - CH₂CH₂NHAc und R10 bis R13 für H-Atome stehen.

8. Ein Purpurin, ein Chlorin, ein PurpurinMetallkomplex oder ein Chlorin-Metallkomplex, wie unter 6 beansprucht, in dem R10 bis R13 durch H-Atome belegt sind.

9. Ein Purpurin oder ein Purpurin-Metallkomplex, wie unter 7 beansprucht.

10. Ein Chlorin oder ein Chlorin-Metallkomplex, wie unter 7 beansprucht.

11. Ein Purpurin wie unter 8 beansprucht, in dem die R1, R3, R5 und R7 jeweils Methylreste sind, die R2, R4, R6 und R8 jeweils Ethylreste und R9 ein CH₃CH₂O₂C-Rest sind.

12. Ein Chlorin, wie unter 8 beansprucht, in dem R1, R3, R5 und R7 Methylreste, R2, R4, R6 und R8 Ethylreste und R9 ein CH₃CH₂O₂C-Rest sind.

13. Ein Purpurin, wie unter 8 beansprucht, in dem R1, R3, R5 und R7 Methylreste, R2, R4, R6 und R8 Ethylreste und R9 ein CH₃CH₂O₂C-Rest sind.

14. Ein Chlorin-Metallkomplex, wie unter 8 beansprucht, in dem R1, R3, R5 und R7 Methylreste, R2, R4, R6 und R8 Ethylreste und R9 ein CH₃CH₂O₂C-Rest sind.

15. Ein Chlorin, ein Chlorin-Metallkomplex oder ein Purpurin-Metallkomplex, wie unter 7 beansprucht, in dem die R1 bis R8 für Alkylreste mit 1 bis 4 C-Atomen, mit Ausnahme des t-Butylrestes, in dem R9 für CO₂R', CH₂CO₂R' oder CH₂CH₂CO₂R' stehen, wobei R' ein H-Atom oder ein Alkylrest mit 1 bis 4 C-Atomen ist, mit Ausnahme des t-Butylrestes, und M für Sn oder Zn steht.

16. Ein Purpurin, wie unter 7 beansprucht, in dem die R1 bis R8 für Alkylreste mit 1 bis 4 C-Atomen, mit Ausnahme des t-Butylrestes, und in dem R9 für CO₂R', CH₂CO₂R' oder CH₂CH₂CO₂R'stehen, wobei R' ein H-Atom oder ein Alkylrest mit 2 bis 4 C-Atomen ist, mit Ausnahme des t-Butylrestes.

17. Ein Purpurin-Metallkomplex, wie unter 12 beansprucht, in dem M für Sn oder Zn steht.

18. Ein Chlorin-Metallkomplex, wie unter 13 beansprucht, in dem M für Sn oder Zn steht.

## Revendications

1. Une solution dans un solvant organique d'un soluté qui est une purpurine ayant la structure de la fig. 5, ci-dessous, un complexe métallique d'une purpurine ayant la structure de la fig. 1, ci-dessous, une chlorine ayant la structure de la fig. 6, ci-dessous, un complexe métallique d'une chlorine ayant la structure de la fig. 2, ci-dessous: 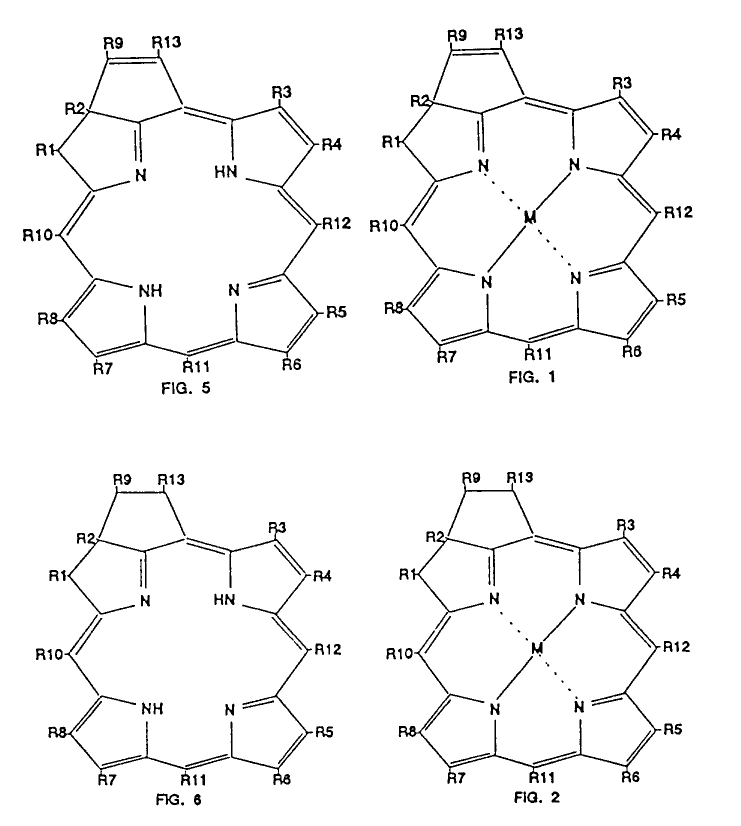 où M est Ag, Al, Ce, Co, Cu, Cr, Dy, Er, Eu, Fe, Ga, Gd, Hf, Ho, In, La, Lu, Mn, Mo, Nd, Pb, Pd, Pr, Pt, Rh, Sb, Sc, Sm, Sn, Tb, Th, Ti, Tl, Tm, U, V, Y, Yb, Zn ou Zr, et chaque R1 à R13 est:
H ou CHO,
un groupe alkyle autre que le t-butyle ayant de 1 à 4 atomes de carbone,
un groupe alkylène ayant de 2 à 4 atomes de carbone,
un radical hydrocarbure aliphatique bivalent ayant de 2 à 4 atomes de carbone où les deux valences du radical sont attachées au même atome de carbone de celui-ci et à un atome de carbone de la purpurine, de la chlorine ou du complexe métallique,
un groupe ayant la formule R₂N(R₃)₂ où R₂ est un radical hydrocarbure aliphatique bivalent ayant de 1 à 4 atomes de carbone, où toute liaison carbone-carbone est une liaison soit simple, soit double, et pas plus de l'une d'entre elles est une liaison double; R₃ est de l'hydrogène ou un groupe alkyle ayant de 1 à 2 atomes de carbone, et les deux groupes R₃ peuvent être identiques ou différents,
un groupe ayant la formule R₂N(R₄)₃A où R₂ est un radical hydrocarbure aliphatique bivalent ayant de 1 à 4 atomes de carbone, où toute liaison carbone-carbone est une liaison soit simple, soit double, et pas plus de l'une d'entre elles est une liaison double; A est un anion physiologiquement acceptable; et R₄ est un groupe alkyle ayant de 1 à 2 atomes de carbone, et les trois groupes R₄ peuvent être identiques ou différents,
un groupe ayant la formule R₂OH où R₂ est un radical hydrocarbure aliphatique bivalent ayant de 1 à 4 atomes de carbone, où toute liaison carbone-carbone est une liaison soit simple, soit double, et pas plus de l'une d'entre elles est une liaison double, ou
CO₂R', CH₂CO₂R' ou CH₂CH₂CO₂R' où R' est de l'hydrogène ou un groupe alkyle autre que le t-butyle ayant de 1 à 4 atomes de carbone,
à condition que pas plus de un R1 à R13 est CHO, un groupe ayant la formule R₂N(R₃)₂, ou un groupe ayant la formule R₂N(R₄)₃A, avec comme condition supplémentaire que seul R1 puisse être un radical hydrocarbure bivalent ayant de 2 à 4 atomes de carbone, où les deux valences du radical sont attachées au même atome de carbone de ce radical et à un atome de carbone de la purpurine, de la chlorine ou du complexe métallique, et où le solvant comporte un liquide organique, et ce solvant est physiologiquement acceptable et présente une concentration adéquate ou peut être dilué à une concentration adéquate pour administration intraveineuse, pour utilisation comme substance pharmaceutique active.

2. Une solution dans un solvant conforme à la revendication 1 utilisée comme substance de détection et de traitement de tumeurs.

3. Une suspension ou émulsion aqueuse d'une solution selon la revendication 1 d'une purpurine ayant la structure de la fig. 5.

4. Une suspension ou émulsion aqueuse d'une solution selon la revendication 1 d'un complexe métallique d'une purpurine ayant la structure de la fig. 1.

5. Une suspension ou émulsion aqueuse d'une solution selon la revendication 1 d'une chlorine ayant la structure de la fig. 6.

6. Une suspension ou émulsion aqueuse d'une solution selon la revendication 1 d'un complexe métallique d'une chlorine ayant la structure de la fig. 2.

7. Une purpurine ou une chlorine ayant la structure de la fig. 5 ou 6, ci-dessous, ou un complexe métallique d'une purpurine ou d'une chlorine ayant la structure de la fig. 1 ou 2, ci-dessous, 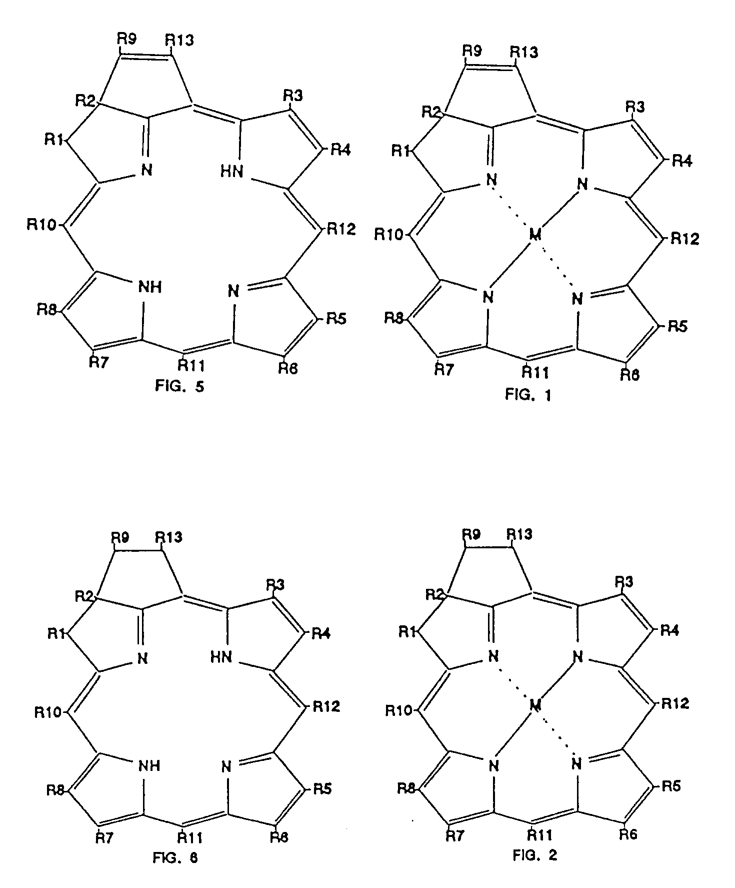 où M est Ag, Al, Ce, Co, Cr, Cu, Dy, Er, Eu, Fe, Ga, Gd, Hf, Ho, In, La, Lu, Mn, Mo, Nd, Pb, Pd, Pr, Pt, Rh, Sb, Sc, Sm, Sn, Tb, Th, Ti, Tl, Tm, U, V, Y, Yb, Zn ou Zr, et chaque R1 à R13 est:
H ou CHO,
un groupe alkyle autre que le t-butyle ayant de 1 à 4 atomes de carbone,
un groupe alkylène ayant de 2 à 4 atomes de carbone,
un radical hydrocarbure aliphatique bivalent ayant de 2 à 4 atomes de carbone où les deux valences du radical sont attachées au même atome de carbone de celui-ci et à un atome de carbone de la purpurine, de la chlorine ou du complexe métallique,
un groupe ayant la formule R₂N(R₃)₂ où R₂ est un radical hydrocarbure aliphatique bivalent ayant de 1 à 4 atomes de carbone, où toute liaison carbone-carbone est une liaison soit simple, soit double, et pas plus de l'une d'entre elles est une liaison double; R₃ est de l'hydrogène ou un groupe alkyle ayant de 1 à 2 atomes de carbone, et les deux groupes R₃ peuvent être identiques ou différents,
un groupe ayant la formule R₂N(R₄)₃A où R₂ est un radical hydrocarbure aliphatique bivalent ayant de 1 à 4 atomes de carbone, où toute liaison carbone-carbone est une liaison soit simple, soit double, et pas plus de l'une d'entre elles est une liaison double; A est un anion physiologiquement acceptable; et R₄ est un groupe alkyle ayant de 1 à 2 atomes de carbone, et les trois groupes R₄ peuvent être identiques ou différents,
un groupe ayant la formule R₂OH où R₂ est un radical hydrocarbure aliphatique bivalent ayant de 1 à 4 atomes de carbone, où toute liaison carbone-carbone est une liaison soit simple, soit double, et pas plus de l'une d'entre elles est une liaison double, ou
CO₂R', CH₂CO₂R' ou CH₂CH₂CO₂R' où R' est de l'hydrogène ou un groupe alkyle autre que le t-butyle ayant de 1 à 4 atomes de carbone,
à condition que pas plus de un R1 à R13 est CHO, un groupe ayant la formule R₂N(R₃)₂, ou un groupe ayant la formule R₂N(R₄)₃A, avec comme condition supplémentaire que seul R1 puisse être un radical hydrocarbure bivalent ayant de 2 à 4 atomes de carbone, où les deux valences du radical sont attachées au même atome de carbone de ce radical et à un atome de carbone de la purpurine, de la chlorine ou du complexe métallique, avec comme condition finale que quatre purpurines ayant la structure de la fig. 5 sont exclues de la revendication, c'est-à-dire deux où R1 à R8 sont de l'éthyle, R9 est soit CHO ou CO₂CH₃, et R10 à R13 sont H, et deux où R1, R4, R6 et R8 sont CH₃, R2 est CH₂CH₂CO₂CH₃, R3 et R9 sont CO₂CH₃, R5 est CH₂CH₃, R7 est soit -CH=CH₂ ou -CH₂CH₂NHAc, et R10 à R13 sont H.

8. Une purpurine, une chlorine, un complexe métallique de purpurine ou un complexe métallique de chlorine selon la revendication 6 où chaque R10 à R13 est de l'hydrogène.

9. Une purpurine ou un complexe métallique d'une purpurine selon la revendication 7

10. Une chlorine ou un complexe métallique d'une chlorine selon la revendication 7.

11. Une purpurine selon la revendication 8 où chaque R1, R3, R5 et R7 est du méthyle, chaque R2, R4, R6 et R8 est de l'éthyle, et R9 est CH₃CH₂O₂C-.

12. Une chlorine selon la revendication 8 où chaque R1, R3, R5 et R7 est du méthyle, chaque R2, R4, R6 et R8 est de l'éthyle, et R9 est CH₃CH₂O₂C-.

13. Un complexe métallique de purpurine selon la revendication 8 où chaque R1, R3, R5 et R7 est du méthyle, chaque R2, R4, R6 et R8 est de l'éthyle, et R9 est CH₃CH₂O₂C-.

14. Un complexe métallique de chlorine selon la revendication 8 où chaque R1, R3, R5 et R7 est du méthyle, chaque R2, R4, R6 et R8 est de l'éthyle, et R9 est CH₃CH₂O₂C-.

15. Une chlorine, un complexe métallique de chlorine ou un complexe métallique de purpurine selon la revendication 7 où chaque R1 à R8 est un groupe alkyle autre que le t-butyle ayant de 1 à 4 atomes de carbone, R9 est CO₂R', CH₂CO₂R' ou CH₂CH₂CO₂R' où R' est de l'hydrogène ou un groupe alkyle autre que le t-butyle ayant de 1 à 4 atomes de carbone, et M est Sn ou Zn.

16. Une purpurine selon la revendication 7 où chaque R1 à R8 est un groupe alkyle autre que le t-butyle ayant de 1 à 4 atomes de carbone, et R9 est CO₂R', CH₂CO₂R' ou CH₂CH₂CO₂R' où R' est de l'hydrogène ou un groupe alkyle autre que le t-butyle ayant de 1 à 4 atomes de carbone.

17. Un complexe métallique de purpurine selon la revendication 12 où M est Sn ou Zn.

18. Un complexe métallique de chlorine selon la revendication 13 où M est Sn ou Zn.
